# EUROPEAN PATENT SPECIFICATION

(11) **EP 1 343 476 B1**
(45) Date of publication and mention of the grant of the patent: **28.05.2008**
(21) Application number: 01998209.9
(22) Date of filing: 03.12.2001
(51) Int. Cl.: A61K 9/127, A61K 39/00

(54) **PREPARATION OF LARGE LIPOSOMES BY INFUSION INTO PEG**
HERSTELLUNG GROSSER LIPOSOME DURCH INFUSION IN PEG
PREPARATION DE LIPOSOMES DE GRANDE TAILLE PAR PERFUSION DANS DU PEG

(30) Priority: 01.12.2000 US 250124 P
(43) Date of publication of application: 17.09.2003
(73) Proprietor: Biomira Inc., Edmonton, Alberta T6N 1H1 (CA)
(72) Inventor: BONI, Lawrence, Monmouth Junction, NJ 08852 (US); WU, Fangjun, West Orange, NJ 07052 (US); FENNIMORE, Roy, Lawrenceville, NJ 08648 (US); BATENJANY, Michael, M, Hamilton, NJ 08619 (US)
(74) Representative: Mallalieu, Catherine Louise
(86) International application number: PCT/US2001/045142
(87) International publication number: WO 2002/043699

(56) References cited:
- WO-A-90/11780
- WO-A-99/36056
- UENO M ET AL: "The preparation of single bilayer liposome by injection method and the effect of some additives on the size distribution" YAKUGAKU ZASSHI, vol. 105, no. 3, 1985, pages 224-231, XP008006097 ISSN: 0031-6903
- PONS M ET AL: "Liposomes obtained by the ethanol injection method" INTERNATIONAL JOURNAL OF PHARMACEUTICS, vol. 95, 1993, pages 51-56, XP000960991 ISSN: 0378-5173
- BATRZI S ET AL: "Single bilayer liposomes prepared without sonication" BIOCHIMICA ET BIOPHYSICA ACTA, vol. 298, 1973, pages 1015-1019, XP000878589 ISSN: 0006-3002 cited in the application

## Description

### SUMMARY OF THE INVENTION

This invention provides a method of preparing large liposomes with high entrapment capacity for pharmaceutical substances.

### BACKGROUND OF THE INVENTION

Liposomes are artificial vesicles that are completely closed lipid bilayer membranes containing an entrapped aqueous volume. Liposomes can differ in size. They can also differ in terms of lipid composition and structural organization corresponding to unilamellar (possessing a single membrane bilayer) or multilamellar (onion-like structure characterized by multiple membrane layers, each separated from the next by an aqueous layer).

Liposomes have been widely used as vehicles for drug delivery. Types of liposomes used in drug delivery include small unilamellar vesicles (SUVs), large unilamellar vesicles (LUVs), and multilamellar vesicles (MLVs). One of the most important characteristics that governs their use is vesicle size, which affects the rate of clearance from blood and organ distribution. Liposomes between 25 nm and 10 µm (microns) have been formulated by a number of techniques to entrap pharmaceuticals.

Based on experimental comparisons between large and small liposomes, large liposomes are cleared from the blood more rapidly than small liposomes (Poste et al., 1984; Allen et al., 1983; Hunt et al., 1979). Rapid clearance is an important feature that will alter the biodistribution of liposomal entrapped drugs. Comparative studies showing greater accumulation of large liposomes have been observed in the lung (Hunt et al., 1979; Abra et al., 1984; Jackson, 1980; Fidler et al., 1980), the liver (Harashima et al., 1992; Jackson, 1980; Allen et al., 1983), and spleen (Moghimi et al., 1991; Jackson, 1980). Large liposomes have been shown to favor uptake by Kupffer cells while small liposomes have a greater affinity to parenchyma cells (Rahman et al., 1982). Small liposomes were also shown to be capable of crossing barriers such as the sinusoidal endothelium (Poste et al., 1984). Thus, depending on the target site and the pharmacokinetics of the entrapped drug, large or small liposomes may have distinct advantages. Ueno et al., Yakugaku Sasshi, 105(3):224-231 (1985), describe the preparation of single bilayer liposomes by injecting an ethanol solution into egg lecithin (*i*.*e*., a lipid component).

WO 90/11780 A discloses a process for preparing a lipid suspension of defined particle size encapsulating a useful compound. The effect of various process parameters on the final liposome products is shown.

Limitations in the current methods of making large liposomes, however, complicate their manufacture. Specifically, organic solvents (*e*.*g*., chloroform) that are commonly used to ensure uniform mixing of lipid components as well as lipophilic pharmaceuticals, are highly toxic. Moreover, removal of organic solvent from the pharmaceutical preparations is difficult and tedious. Scale-up production represents a further problem for the preparation of large liposomes. In addition, commonly used methods, such as ethanol infusion, are inappropriate since the resulting product consists primarily of vesicles in the submicron range (Batzri and Korn, 1973).

To overcome the above-mentioned obstacles, there is an ongoing need to prepare liposomes that are free of highly toxic organic solvents and uniformly sized in the micron range, using methods that are amenable for scaled-up production.

### SUMMARY OF THE INVENTION

One aim of the invention is to provide a method of preparing large liposomes that does not require the use of highly toxic organic solvents, is cost-efficient, and is fully compatible with large-scale manufacturing. It is also an object of the invention to provide a method of preparing uniformly large liposomes, in the micron size range, that exhibit a high entrapment of both lipophilic and water-soluble pharmaceuticals. Another aim of the invention is to provide a method that can be applied to the manufacture of liposomal cancer vaccines suitable for biomedical applications.

In accomplishing these and other aims, there has been provided in accordance with one aspect of the present invention a method of preparing a micron-sized liposome comprising:
(a) providing a first solution comprising a lipid component in a water-miscible alcohol or an alcohol/water mixture wherein the lipid concentration of the first solution is at least 10 mg/ml,
(b) providing an aqueous second solution of water-soluble polymer wherein the polymer is polyethylene glycol (PEG), and the polymer concentration is at least 25% (w/w),
(c) infusing the first solution directly into the second solution thereby forming a liposome suspension, and
(d) removing the alcohol and polymer from the suspension to produce the desired micron-sized liposome.

In a preferred embodiment, the lipid component comprises a mixture of at least one phospholipid and a sterol such as cholesterol.

In another embodiment, a method of preparing large liposomes further comprises adding a pharmaceutical substance to either the first solution comprising a mixture of at least one phospholipid and sterol or to the aqueous solution comprising a water-soluble polymer. The pharmaceutical substance may include a protein, a peptide, or a biological.

In a preferred embodiment, the method produces a liposome comprising a tumor antigen and said liposome is suitable for the manufacture of a cancer vaccine. The liposome produced by ths method may further comprise at least one phospholipid, cholesterol, Lipid A.

In yet another preferred embodiment, the method produces a liposome comprising an infectious disease agent antigen and said liposome is suitable for the manufacture of an infectious disease agent vaccine. Such infectious disease agent antigens can be, for example, bacteria, viruses, parasites, or a mixture thereof.

### BRIEF DESCRIPTION OF THE DRAWINGS

FIG. 1. Requirements of varying PEG 6,000 concentrations by different lipid mixtures for the generation of large liposomes.
FIG. 2. Effect of infusion temperature on mean liposome size using 50% PEG 6,000.
FIG. 3. Effect of infusion temperature on mean liposome size using 50% PEG 20,000.
FIG. 4. Effect of ionic strength of the PEG solution on the final liposome size and its dependence on lipid composition.
FIG. 5. Dependence of volume and ionic strength of the PEG solution on liposome size.
FIG. 6. Variable cholesterol content can differentially affect the effects of PEG and saline infusions on liposome size.
FIG. 7A. Proliferation of lymph node T cells after vaccination with BLP-25 formulations.
FIG. 7B. Interferon-γ production by T cells from mice injected with BLP-25 formulations.

### DETAILED DESCRIPTION OF THE INVENTION

The present invention relates to a novel method for preparing large liposomes with high entrapment of a pharmaceutical by infusion of a lipid-containing alcohol solution directly into an aqueous polymeric solution of polyethylene glycol (PEG) solution.

A prior art method demonstrated that small unilamellar vesicles (SUVs) will fuse to large multilamellar vesicles (MLVs) in the presence of 25-50% PEG, MW 6,000 (Hui et al., 1981; Boni et al., 1981 ; Boni et al., 1984). The resulting large liposomes, typically in the micron range, were multilayered and exhibited a high entrapment of both lipophilic and water-soluble pharmaceuticals. Unlike the prior art method, the present invention circumvents the step of preparing SUVs by directly infusing a lipid/alcohol solution into an aqueous polymer solution. Furthermore, PEG, in particular, is a nontoxic water-soluble polymer, available at low cost, and is FDA-approved for internal consumption (Harris, 1997; Working et al., 1997).

In one aspect of the invention, a first solution comprising a lipid component and a water-miscible alcohol or an alcohol/water mixture is provided. The lipid/alcohol solution may be an emulsion, however the lipid component usually comprises a mixture of at least one phospholipid and a sterol. Preferred phospholipids are capable of forming liposomes and include phospholipids such as phosphatidylcholine (PC; lecithin). Suitable phospholipids also include phosphatidic acid (PA), phosphatidylglycerol (PG), phosphatidylethanolamine (PE), and phosphatidylserine (PS). Other suitable phospholipids further include dipalmitoylphosphatidylcholine (DPPC), distearoylphosphatidylcholine (DSPC), dimyristoylphosphatidylcholine (DMPC), dipalmitoylphosphatidylglycerol (DPPG), distearoylphosphatidylglycerol (DSPG), dimyristoylphosphatidylglycerol (DMPG), dipalmitoylphosphatidic acid (DPPA); dimyristoylphosphatidic acid (DMPA), distearoylphosphatidic acid (DSPA), dipalmitoylphosphatidylserine (DPPS), dimyristoylphosphatidylserine (DMPS), distearoylphosphatidylserine (DSPS), dipalmitoylphosphatidylethanolamine (DPPE), dimyristoylphosphatidylethanolamine (DMPE), and distearoylphosphatidylethanolamine (DSPE). The preferred sterol is cholesterol. Further suitable ingredients of the lipid component are oils, saturated and unsaturated lipid mixtures, glycolipids, and other lipid adjuvants, such as monophosphoryl lipid A (MPLA) or Lipid A. Preferred alcohols include lower alkanols such as ethanols, methanols, propanols, and butanols (*i.e*., *iso*-butanol, *sec*-butanol, and *tert*-butanol).

To prepare the first solution, a powdered lipid mixture can be dissolved in a minimal amount of alcohol (typically ethanol) with or without water. The water content preferably is less than about 10% of the total volume. The lipid concentration usually is at least about 30 mg/mL, but may be between 5-80 mg/mL. Cholesterol percentage, preferably 0-50 Mol% cholesterol, can be adjusted for optimal entrapment, control of liposome size, and pharmaceutical activity, as determined empirically by the following examples. Optionally, one or more pharmaceutical substances may be added at this point to the lipid/alcohol mixture. Suitable pharmaceutical substances used in the invention may include proteins, peptides, and biologicals.

Preferred proteins include an antibody or an antibody fragment. Preferred peptides include lipopeptides, glycopeptides, and glycolipopeptides. A biological may also refer to DNAs or RNAs, such as intact DNA or mRNA molecules, as well as nucleosides, nucleotides, and oligo and polynucleotides. In addition, biologicals may refer to synthetic or natural organic molecules, vitamins, steroids, immunomodulators, drugs such as antibiotics, anti-tumor agents, anti-inflammatory agents, anti-angiogenesis agents, chemokines, drugs acting on the central nervous system, adjuvants, and infectious disease agent antigens, such as bacteria, viruses, and parasites.

A brief sonication bath above the main phase transition temperature (Tₘ) of the lipid can be used to accelerate dissolution. The main phase transition temperature in the present invention refers to the temperature wherein a typical hydrated lipid transitions from a gel phase with highly ordered hydrocarbon chains to a liquid crystalline phase with disordered hydrocarbon chains. The resultant clear alcohol solution may be sterilized, e.g., by filtration through a 0.22µm (micron) filter.

Second, a separate solution of the water-soluble polymer, polyethylene glycol (PEG), is provided, for example, by mixing the polymer with water under constant stirring. A preferred water soluble polymer is typically, PEG 1,000 (M.W. 1,000), PEG 6,000 (M.W. 6,000), or PEG 20,000 (M.W. 20,000). Polymer concentrations ranging from 10% to about 50% (w/w) are employed. PEGs of high polymerization degree, e.g., PEG 6,000, are employed due to lack of toxicity. Salt content of the PEG solution can be adjusted from 0 to about 10% (w/v), e.g., by adding sodium chloride (NaCI) (preferably between 50 and 154 mM NaCl). This range will be based upon optimal entrapment, resultant liposome size, and pharmaceutical activity. The polymer solution can be sterilized, for example, by filtration (0.22 µm (micron) filter) or by autoclaving. A water-soluble pharmaceutical can be optionally added to the polymer solution.

Third, the sterile lipid/pharmaceutical(s) alcohol solution is directly infused into the polymer/pharmaceutical(s) aqueous solution or mixed by directing opposing streams of the alcohol solution to the PEG solution at predetermined volume ratios (*e*.*g*., 4:1 to 1:4 Lipid/alcohol:PEG (v/v)). Basically, the ratio of the lipid to polymer, average M.W. ofpolymer, polymer concentration, salt content (ionic strength), and temperatures of infusion and incubation are variables in controlling the liposome size, as well as entrapment of a pharmaceutical, as shown in more detail in the Examples below.

Fourth, the resultant mixture is incubated for a time sufficient to obtain a uniform suspension. Typical incubation times run from at least about 15 minutes to about 24 hours at a temperature ranging from about 4 °C to about 55 °C with intermittent mixing. The mixing and incubation temperature are preferably above the Tₘ of the lipid mixture to insure good mixing of the components. To ensure the stability of the entrapped pharmaceutical(s), extended exposure to high temperature should be avoided.

Fifth, polymer and alcohol typically are removed by washing the liposomes. One method of washing is by addition of sterile water and centrifugation of the mixture, followed by resuspension of the liposomal pellet with sterile water or a physiologically acceptable solution, like normal saline (0.9% (w/v) NaCl). The centrifugation and resuspension are repeated until satisfactory removal of the polymer and alcohol are achieved. The final pellet is resuspended in a physiologically acceptable solution to reach the desired final lipid concentration. This concentration is dependent on the formulation, but is preferably between about 15 to 50 mg/mL bulk lipid (w/v). Washing can also be achieved by standard diafiltration methods under aseptic conditions. The final liposomes are multilamellar vesicles (MLVs) with pharmaceuticals incorporated. The typical average size of the MLVs ranges from one to eight microns depending on the parameters employed.

The instant invention of making larger multilamellar liposomes is simpler, safer, and different from the prior art that is based on the infusion of lipids dissolved in ethanol (Batzri and Korn, 1973; and Kremer et al., 1977) or a water-immiscible solvent (Coe et al., 1996) into water or aqueous buffer. The latter procedures yield predominantly small unilamellar vesicles (SUVs) of mean diameter about 25 nm to 200 nm. In addition, the multilamellar vesicles (MLVs) prepared by the instant invention typically have a mean diameter that ranges from one to eight microns, which is larger than those obtained by Coe et al. (1996) or Leigh (1991).

Not wishing to be bound to a particular theory, it is believed that the presence of the polymer causes lipid molecules to coalesce into large multilamellar vesicles. The replacement of alcohol, with the excess bulk water and bound water in the polymer solution, causes the lipid molecules to become immediately hydrated and to spontaneously form a large bilayer structure. The small volume of "free" water within the MLVs forces a high entrapment of the pharmaceutical in either the bilayer hydrophobic core or the aqueous space between the lamellae. The size of the resultant MLVs and their high entrapment of pharmaceuticals make them good candidates for delivery of therapeutic agents.

Liposomes prepared using methods according to the above invention can be used in a wide variety of applications, particularly in inducing potent *in vivo* immunity to incorporated antigen. Liposomes are now employed in numerous immunization procedures. The present liposomes may be substituted into those procedures. Specifically, this method has also been applied to the manufacture of vaccines.

Nearly any type of antigen may be incorporated in any of the present liposomes, *e*.*g*., for the manufacture of vaccines, including tumor antigens. DNA and RNA encoding antigens may also be used. Tumor antigens may be derived from lung cancers, colon cancers, melanomas, neuroblastomas, breast cancers, ovarian cancers, and the like. A preferred tumor antigen is MUC-1 and its related antigenic peptides and their derivatives. MUC-1 mucin is a high molecular weight glycoprotein with a protein core consisting of tandem repeats of a 20-amino acid sequence and highly-branched carbohydrate side chains. Underglycosylated MUC-1 protein is secreted and abundantly over-expressed in many human adenocarcinomas, such as breast, colon, lungs, ovarian, and pancreatic cancers. An enhanced level of MUC-1 expression is associated with high metastatic potential and poor prognosis. Thus, MUC-1 is a clinically significant marker for these cancers. Particularly useful antigenic MUC-1 peptide derivatives are based on the 20-amino acid repeat sequence, which may be lipid-modified and/or carbohydrate-modified according to standard methods.

A preferred MUC-1 peptide is BP1-148, a 25-mer antigenic MUC-1-derived lipopeptide, as given by the following sequence:
NH₂-[STAPPAHGVTSAPDTRPAPGSTAPP(K-palmitoyl)G]-COOH
Two non-MUC-1 amino acids are added as a scaffold for attaching the lipid tail, making a total of 27 amino acids. BP1-148 has a palmitoyl group at the epsilon amino group of the C-terminal lysine. This lipopeptide is the immunogen in a liposomal vaccine (BLP-25) currently in clinical trials for several different types of cancer. Forms of BP1-148, both with and without a lipid tail, are described in Agrawal, B. et al., U.S. Patent No. 6,600,012, and Boni. L. et al., U.S. Application Publication No. 20020051813.

We describe a liposomal vaccine formulation that may also comprise one or more immunomodulators. An immunomodulator is any substance that alters an immune. response, and preferably, stimulates a response to antigen. Typical immunomodulators include but are not limited to adjuvants such as monophosphoryl Lipid A (MPLA), Lipid A, synthetic Lipid A and variants, muramyl dipeptides, CpG oligo's, and lipoteichoic acid. MPLA has been shown to serve as an effective adjuvant to cause increase presentation of lysosomal antigen by the antigen presenting cells to specific T lymphocytes (Alving, C.R. 1993). Other immunomodulators include cytokines and lymphokines, especially intetieddn-2 (IL-2), chemokines, and costimulatory molecules, especially CD28, CD40, CD40 Ligand, CD80, and CD86.

The following are examples of the present process, using infusion into PEG. They are given to indicate the many parameters that can be varied to help manufacture an optimal liposomal pharmaceutical.

### EXAMPLES

### Example 1 Effects of water and PEG infusion methods on liposome size.

This example demonstrates that a liposome of a larger mean liposome size can be obtained by direct infusion of the lipid mixtures into an aqueous PEG solution. A smaller size liposome was obtained with water as the infusion media.

A lipid formulation (17.44 mg of DPPC, 2.18 mg of DMPG, and 10.4 mg of cholesterol (50 Mol%)) was dissolved in 0.78 mL of ethanol and 0.04 mL of deionized water. Sonication was performed briefly at 55 ° C in a bath sonicator. The clear solution was then infused into 3.18 mL of either deionized water or a 50% (w/w) PEG 6,000 in deionized water. The mixtures were held at 25 ° C for 1 hour, then diluted to 50 mL with normal saline (0.9% NaCl (w/v)) and subsequently pelleted by centrifugation for 30 min at 18,000 rpm and 4 °C (Sorvall SS-34 rotor). The pellets were washed three times by resuspension into 50 mL normal saline and centrifuged as described above. The final pellets were resuspended in 1.5 mL normal saline.

The mean liposome size was 0.4 µm for the sample infused in water, whereas the same lipid mixture infused into 50% PEG in water resulted in a mean liposome size of 2.12 µm (Table 1). This shows a marked increase in the mean diameter of liposomes formed as a result of infusion into a PEG solution over those infused in water, keeping other parameters such as lipid composition, hydration solvent, and infusion volume constant.

**Table 1 Sizes of Cholesterol-Containing Liposomes Prepared by Infusion Methods**

| **Example** | **Sample Composition (Molar)** | **Infusion Media** | **Liposome Size (µm)** |
|---|---|---|---|
| 1 | DPPC:CHOL:DMPG 44.1:50.0:5.9 | H₂O | 0.4 |
| 1 | DPPC:CHOL:DMPG 44.1:50.0:5.9 | 50% PEG 6,000 | 2.1 |
| 2 | DPPC:CHOL 50.0:50.0 | 50% PEG 6,000 | 1.4 |

### Example 2 Effect of lipid composition on liposome size.

This example shows that the lipid composition can affect the mean liposome size. The lipid formulation in Example 1 was modified to 19.74 mg of DPPC and 10.4 mg of cholesterol (*i.e*., DMPG was excluded). Liposomes were prepared in the same way as in Example 1, by infusion into 50% PEG 6,000 in water. The resulting mean liposome size was 1.40 µm as compared to 2.12 µm for the PEG infusion formulated in Example 1 (Table 1). This demonstrates the strong dependence of liposome size on the lipid composition.

Cholesterol possesses a planar steroid ring which is conformationally inflexible and water insoluble. Hence, the addition of cholesterol orders the lipid bilayer by restricting the conformational flexibility of the lipid hydrocarbon chain and favors a more planar (less curvature) bilayer. The result is a decrease in bilayer packing defects which decreases passive permeability *(i.e.,* reduces solute leakage) and the formation of larger liposomes (planar).

### Example 3 Requirements in the generation of large liposomes.

This example demonstrates that generation of large liposomes is not solely dependent on the different lipid mixtures being added but it is also dependent on the varying PEG concentrations being used.

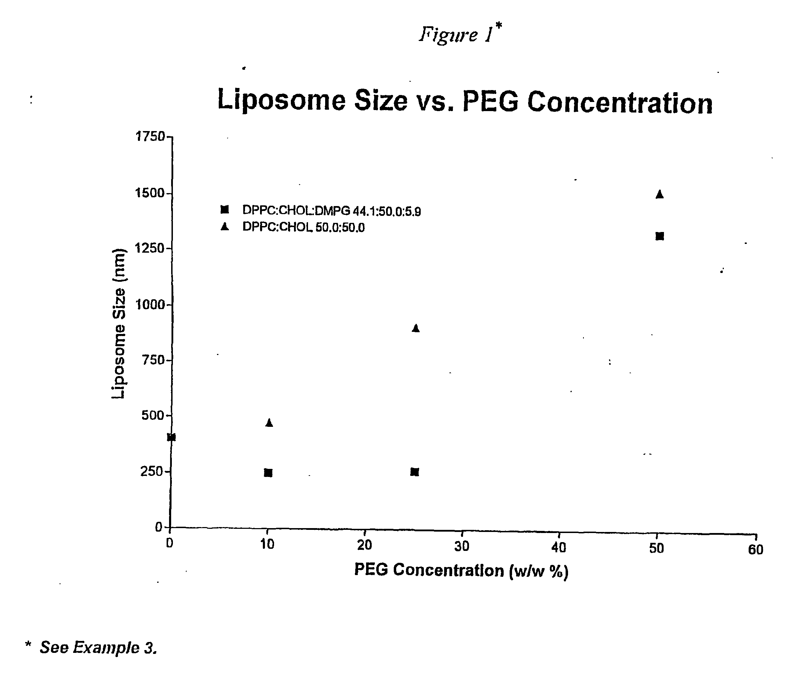

Figure 1 compares mean liposomal sizes obtained when formulations from Examples 1 and 2 were infused into 0,10%, 25%, and 50% (w/w) PEG solutions at 25 ⁰C. As can be seen, different lipid mixtures have different PEG concentration requirements for generating large liposomes.

### Example 4 Effect of infusion temperature on mean liposome size using 50% PEG 6,000.

This example shows that at a higher temperature, a larger liposome can be obtained with 50% PEG 6,000.

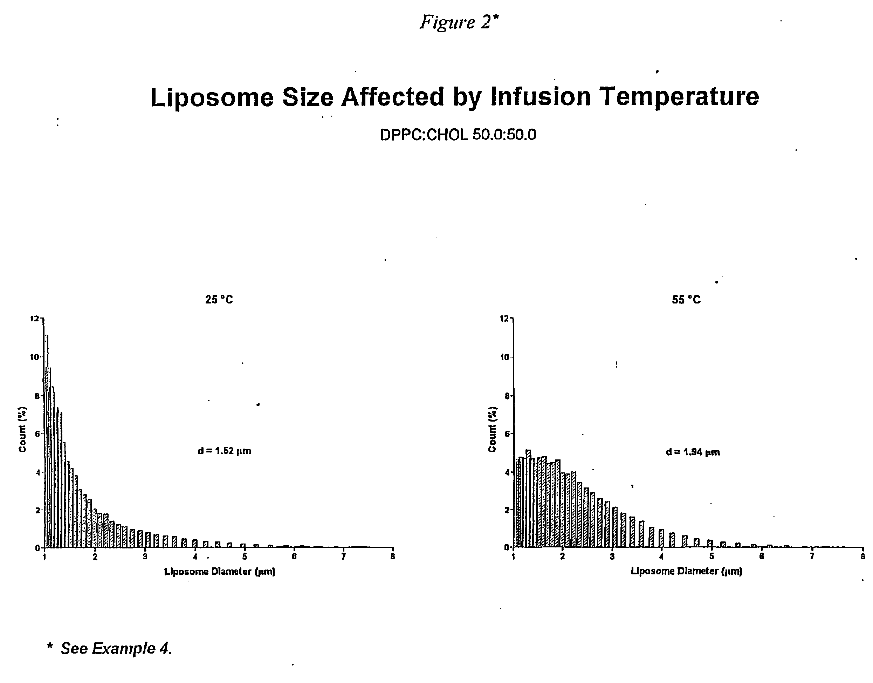

The same preparation was made as in Example 2. The PEG infusion and incubation temperature was 55 ⁰C instead of 25 ⁰C. The mean liposome size obtained was 1.94 µm at 55 ⁰C compared with 1.52 µm at 25 ⁰C (Figure 2). Thus, temperature can be raised to obtain a larger liposome. It can also be lowered to obtain a liposome of a smaller size.

### Example 5 Effect of PEG polymerization on mean liposome size at 25 °C and 55 °C.

This example demonstrates that liposome size is directly dependent on the molecular weight of PEG.

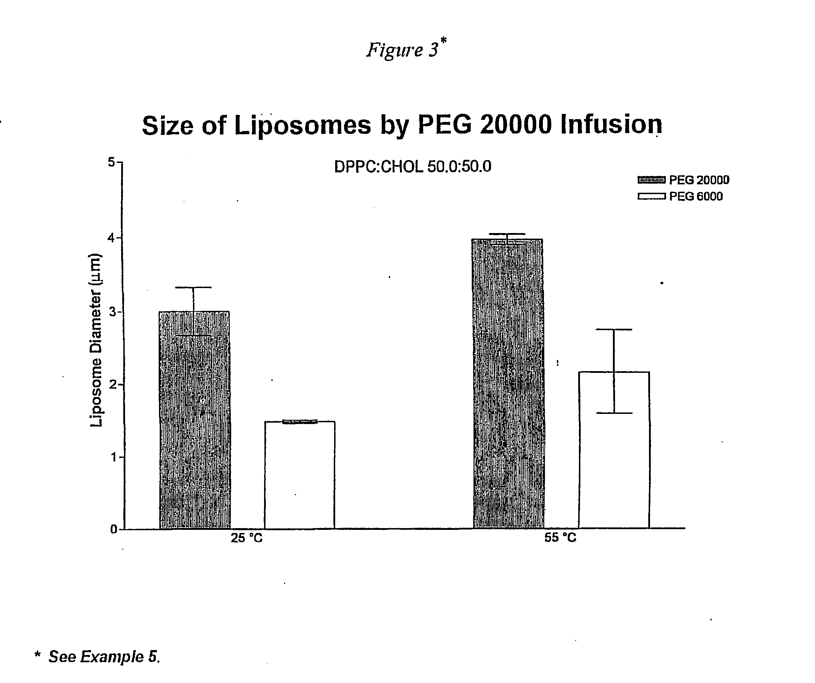

The same preparation was made as in Example 2 with 50% PEG 20,000 instead of PEG 6,000. The mean liposome size was 3.00 µm at 25 ⁰C and 3.97 µm at 55 ⁰C. Comparing these results to the liposome sizes obtained with PEG 6,000 (Figure 3), it appears that infusion into the higher MW PEG yields a larger liposome.

### Example 6 Effect of salt concentration on the final liposome size.

This example illustrates the influence of ionic strength of the PEG solution on the final liposome size and its dependence on lipid composition.

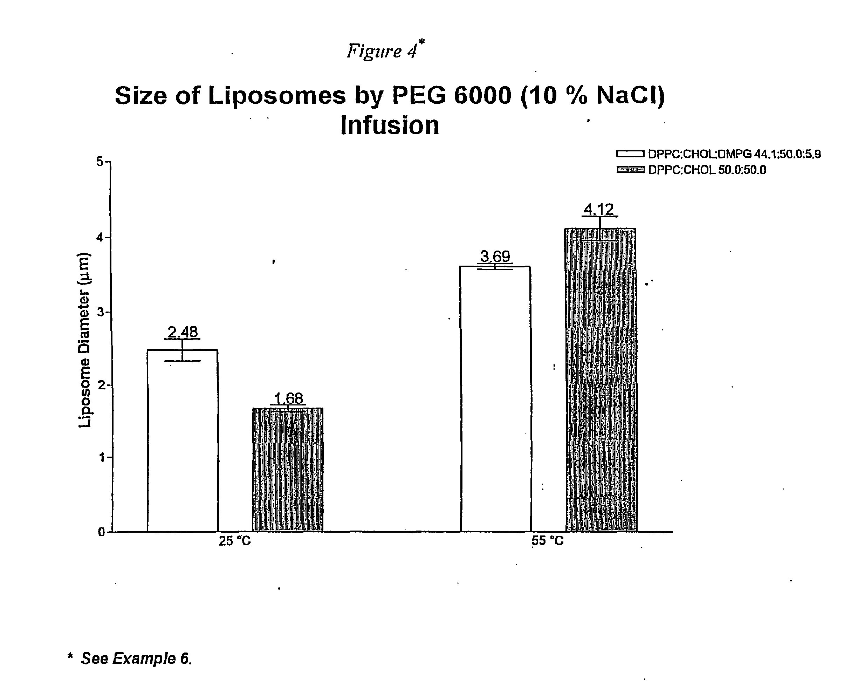

The same preparations were made as in Examples 1 and 2, but infused into 50% PEG 6,000 in 10% NaCl. As shown in Figure 4, a 25 °C infusion into PEG 6,000 in high ionic strength media resulted in mean liposome sizes of 2.48 µm (w/DMPG) and 1.68 µm (w/o DMPG). In contrast, similar preparations infused into PEG 6,000 in low ionic strength solvent gave smaller liposome sizes of 2.12 µm and 1.4 µm, respectively (Table 1). At 55 ⁰C, consistent with the temperature effect noted in Example 4, infusion into PEG 6,000 in high ionic strength media further increased the mean liposome sizes to 3.69 µm (w/DMPG) and 4.12 µm (w/o DMPG). Thus, the ionic strength of the PEG solution can affect the final liposome size.

### Example 7 Effect of infusion into increased volumes of PEG 6,000 in 10% (w/v) NaCl solution on liposome size..

This example shows a decrease in mean liposomal size with infusion into higher volumes of PEG 6,000 in 10% (w/v) NaCl.

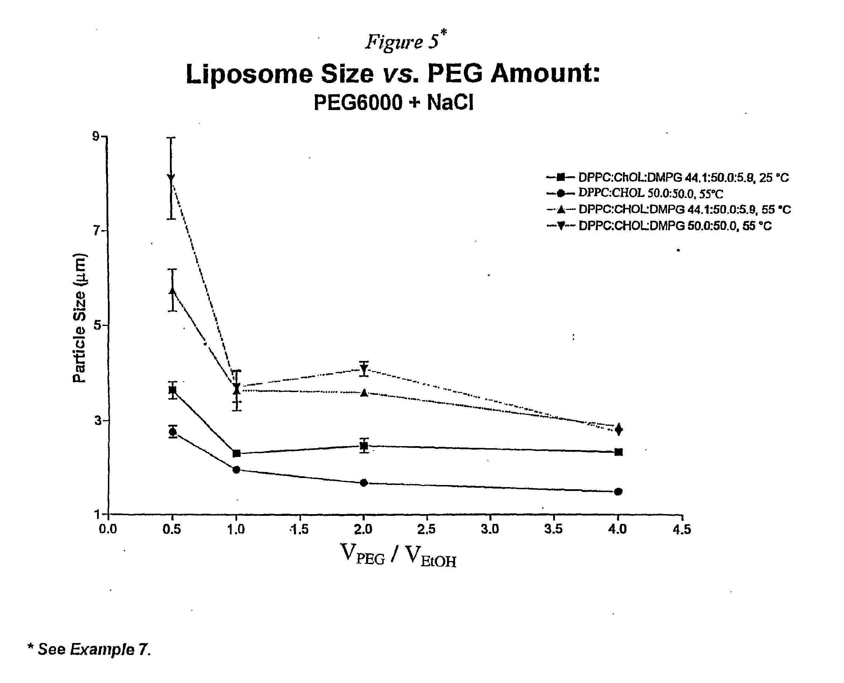

In the manner of Examples 1 and 2, formulations were prepared with and without DMPG in 0.78 mL of ethanol and 0.04 mL of deionized water. These formulations were infused into 3.2 mL, 1.6 mL, 0.8 mL, and 0.4 mL of a 50% PEG 6,000 solution containing 10% NaCl at either 25 °C or 50 °C. Figure 5 exhibits a decrease in mean liposomal size as the ratio of PEG solution:Infusate (v/v) increases. This effect is observed at 25 °C and 50°C and appears independent of DMPG inclusion.

### Example 8 Influence of varying cholesterol content on the effects of PEG and saline infusions on liposome size.

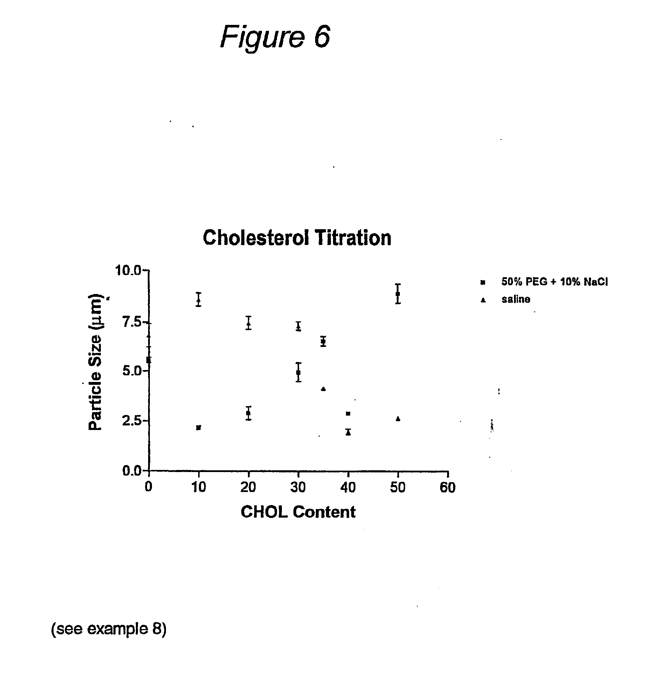

A lipid formulation of 19.75 mg of DPPC with cholesterol varying from 0 mg to 10.4 mg (0 to 50 Mol%) was dissolved in 0.78 mL ethanol and 0.04 mL water. Large liposomes were formed following both PEG (in 10% NaCI) and saline (w/o PEG) infusion. Figure 6 shows that, with increased cholesterol content, saline infusion exhibited a decrease in vesicle size. In contrast, PEG infusion demonstrated an increase in vesicle size with increasing cholesterol content. This indicates that PEG infusion is more appropriate for making larger vesicles at high cholesterol content.

### Example 9 Induction of peptide-specific cellular responses by liposomal vaccines prepared from direct PEG infusion.

The objective of this Example is to illustrate that liposomal vaccines prepared from direct infusion of PEG are capable of stimulating antigenic responses.

Sample FW91699-1 was prepared by infusion of an ethanol solution containing DPPC, cholesterol, Lipid A, and BP1-148 into 50% (w/w) PEG 6,000 in 10% (w/v) NaCl. Sample FW91699-2 was prepared in a similar manner but with infusion into PEG 20,000 in water. The PEG was removed by washing with normal saline followed by centrifugation to obtain the pelleted MLVs. These were compared to a control BLP25 vaccine formulation, BUSA 27, currently in clinical trials. BUSA27 was prepared by first forming LUVs by ethanol infusion, followed by lyophilization and reconstitution in sterile normal saline solution to make MLVs. BUSA 27 also contains DPPC, cholesterol, Lipid A, and BP1-148, but in addition has 5.9 Mol% DMPG.

Each formulation was injected subcutaneously into the inguinal region of C57BL/6 mice. Nine days after injection, spleens and lymph nodes were removed and dissociated into cells. Cells were cultured for 72 hours with peptide BP1-151, a nonpalmitated version of BP1-148, with the negative control peptide BP-072 or with medium. Proliferation of lymph node cells was determined by Alamar Blue assay and release of IFN-y was determined by a capture Ab assay.

Table 2 shows that the liquid formulations, FW91699-1 and FW91699-2, induced peptide-specific (BP1-151) immune responses in mice.

**Table 2 Proliferation and IFN-γ Results**

| Proliferation Assay | | | | |
|---|---|---|---|---|
| Treatment | Optical Density 570/610 nm | | | |
| | Medium | BP-072 | BP1-151 | BP1-151 Peptide Specific Proliferation |
| Saline | 0 | 0.007 | 0 | 0 |
| BUSA 27 | 0.176 | 0.079 | 0.543 | 0.367 |
| FW91699-1 | 0.02 | 0 | 0.410 | 0.390 |
| FW91699-2 | 0.08 | 0 | 0.405 | 0.325 |

| IFN-γ Assay Lymph Nodes | | | | |
|---|---|---|---|---|
| Treatment | Interferon gamma (ng / mL) | | | |
| | Medium | BP-072 | BP1-151 | BP1-151 Peptide Specific IFN-γ Release |
| Saline | 0.7 | 0.7 | 0.7 | 0 |
| BUSA 27 | 3* | 3* | 59* | 56 |
| FW91699-1 | 3* | 3* | 49* | 46 |
| FW91699-2 | 3* | 3* | 51* | 48 |

| IFN-γ Assay Spleen Cells | | | | |
|---|---|---|---|---|
| Treatment | Interferon gamma (ng / mL) | | | |
| | Medium | BP-072 | BP1-151 | BP1-151 Peptide Specific IFN-γ Release |
| Saline | 0.7 | 0.7 | 0.7 | 0 |
| BUSA 27 | 3* | 3* | 114* | 111* |
| FW91699-1 1 | 3* | 3* | 90* | 87* |
| FW91699-2 | 3* | 3* | 85* | 82* |

| | | | | |
|---|---|---|---|---|
| *1:5 dilution all the remaining supernatants were tested at a 1:2 dilution. | | | | |

### Example 10 Proliferation of T Cells and γ-interferon production by different BLP-25-like liposomal vaccine formulations.

This example provides further evidence that BLP-25-like liposomal vaccines formulated from direct PEG infusion can stimulate cellular immune responses by inducing T cell proliferation and IFN-γ production.

Four liquid formulations of BLP-25 vaccine prepared by the PEG-infusion method were manufactured by controlling temperature, infusion rate, and mixing speeds. These were compared to standard preparations denoted BUSA 014 and BUSA 26, two additional lots prepared like BUSA 27 (see Example 9). Both standards are manufactured following the procedure for BUSA 27 and contain DPPC, cholesterol, Lipid A, BLP-148, and 5.9 Mol% DMPG. Infusion was into saline alone or 50% PEG 6,000 in 10% NaCl. The components of the formulations were DPPC (11.6 mg/mL), cholesterol (6.9 mg/mL), BP-148 (400 µg/mL), and Lipid A (200 µg/mL), with and without DMPG as noted in Table 3. These formulations were washed by diafiltration at a constant inlet pressure of 103-138 kPa (15-20 psi) against sterile-filtered phosphate buffered saline (10 mM sodium phosphate, 0.9% (w/v) NaCl, pH 6.6). Infusion into PEG and inclusion of DMPG both contributed to a higher recovery of the BP1-148 peptide (Table 3).

**Table 3 Infusion Into Different Media**

| Liquid Formulations | Mixing Medium | DMPG (1.5 mg/mL) | Recovery of BP1-148 (µg/mL) |
|---|---|---|---|
| BLP-25-1 | normal saline (0.9% NaCl) | + | 204 |
| BLP-25-2 | 50% PEG in 10% NaCl | + | 318 |
| BLP-25-3 | normal saline (0.9% NaCl) | - | 123 |
| BLP-25-4 | 50% PEG in 10% NaCl | - | 243 |

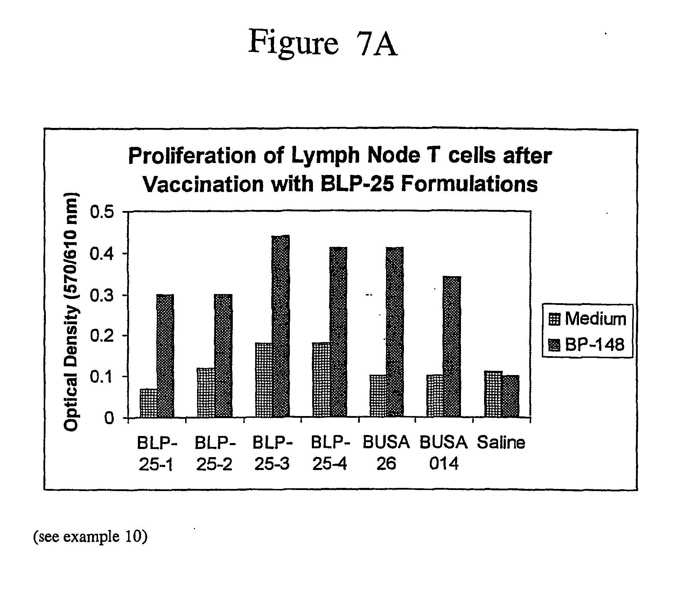

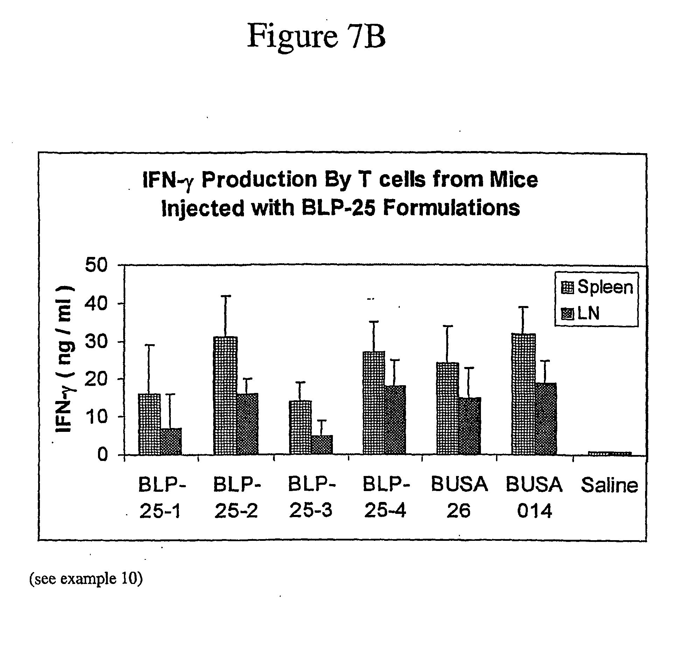

These formulations were injected into C57BL/6 mice (subcutaneous, inguinal region) to determine their potency for induction of peptide-specific immune responses (proliferation of T cells and IFN-γ production). Figure 7A demonstrates that all four formulations induced a BP1-148 specific proliferative response in T cells from the draining lymph nodes which was comparable to that induced by standard preparations BUSA 26 and BUSA 014. Figure 7B shows that all four liquid formulations and the standard preparations, BUSA 26 and BUSA 014, also primed T cells to produce significant amounts of IFN-γ in response to restimulation in vitro with BP1-148. The amount of IFN-γ released by the cells roughly correlated with the recovery of the BP1-148 peptide in the individual formulations (Table 3).

### REFERENCES

Abra, R.M., Hunt, C.A. and Lau, D.T. (1984) Liposome disposition in vivo VI: delivery to the lung. J. Pharm. Sci. 73:203-206.
Angrawal, B. et al., (1988) U.S. Patent No. 6,600,012.
Allen, T.M., and Everest, J.M. (1983) Effect ofliposome size and drug release properties on pharmacokinetics of encapsulated drug in rats. J. Pharm. Exp. Therapeutics 226:539-544.
Alving, C.R (1993) Lipopolysaccharide, lipid A, and liposomes containing lipid A as immunologic adjuvants. Immunobiology 187:430-446.
Batzri, S., and Korn, E.D. (1973) Single bilayer liposomes prepared without sonication. Biochim. Biophys. Acta. 298:1015-1019.
Boni, L.T., Stewart, T.P., Alderfer, J.L., and Hui, S.W. (1981) Lipid-polyethylene glycol interactions: I. Induction of fusion between liposomes. J. Membrane Biol. 62:65-70.
Boni, L.T., Hah, J.S., Hui, S.W., Mukherjee, P., Ho, J.T., and Jung, C.Y. (1984) Aggregation and fusion of unilamellar vesicles by poly(ethylene glycol). Biochim. Biophys. Acta 775:409-418.
Boni, L. et al., (1999) U.S. Application Publication No. 20020051813.
Coe, R.M., Edgerly-Pflug, L., Boni, L., Portnoff, J., and Sharma, M.L. (1996) Unites States Patent 5,540,936.
Fidler, I.J., Ras, A., Fogler, W.E., Kirsh, R., Bugelski, P., and Poste, G. (1980) Design of liposomes to improve delivery of macrophage-augmenting agents to alveolar macrophages. Cancer Res. 40:4460-4466.
Gregoriadis, G., Neerunjun, D.E., and Hunt, R. (1977) Life Sci. 21:357.
Harashima, H., Ohnishi, Y., and Kiwada, H. (1992) In vivo evaluation of the effect of the size and opsonization of the hepatic extraction of liposomes in rats: an application of the Oldendorf method. Biopharm. Drug Disp. 13:549-553.
Harris, J.M. (1992) Introduction to the biotechnical and biomedical applications of poly(ethylene glycol). In: Poly(ethylene glycol) chemistry, biotechnical and biomedical applications, ed. Harris, J.M., pp. 1-14, Plenum Press, New York.
Heath, T.D., Lopez, N.G., and Papahadjopoulos, D. (1985) The effects of liposome size and surface charge on liposome-mediated delivery of methotrexate-aspartate to cells in vitro. Biochim. Biophys. Acta, 820:74-84.
Hui, S.W., Stewart, T.P., Boni, L.T., and Yeagle, P.L. (1981) Membrane fusion through point defects in bilayers. Science 212:921-923.
Hunt, C.A., Rustum, Y.M., Mayhew, E., and Papahadjopoulos, D. (1979) Retention of cytosine arabinoside in mouse lung following intravenous administration in liposomes of different size. Drug Metab. Dispos. 7:124-128.
Jackson, A.J. (1981) Intramuscular absorption and regional lymphatic uptake of liposome-entrapped inulin. Drug Metab. Dispos. 9:535-540.
Jackson, A.J. (1980) The effect of route of administration of the disposition of inulin Encapsulated in multilamellar vesicles of defined particle size. Res. Comm. Chem. Path. Pharm. 27:293-304.
Kremer, J.M., Esker, M.W., Pathmamanoharan, C., Wiersema, P.H. (1977) Vesicles of variable diameter prepared by a modified injection method. : Biochemistry 17:3932-5.
Leigh, S. (1991) United States Patent 5,053,217.
Loughrey, H.C., Wong, K.F., Choi, L.S., Cullis, P.R. and Bally, M.B. (1990) Protein liposome conjugates with defined size distributions. Biochem. Biophys. Acta 1028:73-81.
Moghimi, S.M., Porter, C.J., Muir, I.S., Illum, L., and Davis, S.S. (1991) Non-phagocytic uptake of intravenously injected microspheres in rat spleen: influence of particle size and hydrophilic coating. Biochem. Biophys. Res. Commun. 177:861-866.
Poste, G., Kirsh, R., and Koestler, T. (1984) The challenge of liposome targeting in vivo. In: Liposome Technology, ed., G. Gregoriades, Volume III, pp. 1-28, CR. Press.
Proffitt, R.T., Williams, L.E., Presant, C.A., Tin, G.W., Uliana, J.A., Gambe, R.C., and Baldeschwieler, J.D. (1983) Liposomal blockade of the reticuloendothelial system: improved tumor imaging with small unilamellar vesicles. Science 220:502-505.
Rahman, A., Fumagalli, A., Barbieri, B., Schein, P.S., and Casazza, A.M. (1986) Antitumor and toxicity evaluation of free doxorubicin and doxorubicin entrapped in cardiolipin liposomes. Cancer Chem. Pharm. 16:22-27.
Rahman, Y.E., Cerny, E.A., Patel, K.R., Lau, E.H., and Wright, B.J. (1982) Differential uptake of liposomes varying in size and lipid composition by parenchymal and Kupffer cells of mouse liver. Life Sci. 31:2061-2071.
Suzuki, S., Ohta, S., Takashio, K., Nitanai, H., and Hashimoto, Y. (1990) Int. J. Cancer 46:1095-1100.
Working, P.K., Newman, M.S., Johnson, J., and Cornacoff, J.B. (1077) Safety of poly(ethylene glycol) and poly(ethylene glycol) derivatives. In Poly(ethylene glycol) chemistry and biological applications, eds., Harris, J.M. and Zalipsky, S., pp. 45-57. American Chemical Society, Washington, D.C.

## Claims

1. A method of preparing a micron-sized liposome comprising:
(a) providing a first solution comprising a lipid component in a water-miscible alcohol or an alcohol/water mixture wherein the lipid concentration of the first solution is at least 10 mg/ml,
(b) providing an aqueous second solution of water-soluble polymer wherein the polymer is polyethylene glycol (PEG), and the polymer concentration is at least 25% (w/w),
(c) infusing the first solution directly into the second solution thereby forming a liposome suspension, and
(d) removing the alcohol and polymer from the suspension to produce the desired micron-sized liposome.

2. The method of Claim 1, wherein the liposome produced has an average size of from one µm (micron) to eight µm (microns).

3. The method of Claim I or 2, wherein said alcohol and polymer are removed by washing the mixture through centrifugation or diafiltration.

4. The method of any one of Claims 1 to 3, wherein said lipid component is selected from the group consisting of a phospholipid, an oil, and a mixture of saturated and unsaturated lipids.

5. The method of Claim 4, wherein the phospholipid is selected from the group consisting of phosphatidylcholine, phosphatidylglycerol, phosphatidic acid, phosphatidylserine, and phosphatidylethanolamine, dipalmitoylphosphatidylcholine, distearoylphosphatidylcholine, dimyristoylphosphatidylcholine, dipalmitoylphosphatidylglycerol, distearoylphosphatidylglycerol, dimyristoylphosphatidylglycerol, dipalmitoylphosphatidic acid; dimyristoylphosphatidic acid, distearoylphosphatidic acid, dipalmitoylphosphatidylserine, dimyristoylphosphatidylserine, distearoylphosphatidylserine, dipalmitoylphosphatidylethanolamine, dimyristoylphosphatidylethanolamine, and distearoylphosphatidylethanolamine.

6. The method of any one of Claims 1 to 5, wherein said lipid component comprises at least one lipid and a sterol.

7. The method of Claim 6, wherein the sterol is a cholesterol.

8. The method of any one of Claims 1 to 7, wherein the lipid and water-miscible alcohol or the lipid and alcohol/water mixture is an emulsion.

9. The method of any one of Claims 1 to 8, wherein the molecular weight of the polymer is from about 6,000 Dalton to about 20,000 Dalton.

10. The method of any one of Claims 1 to 9, wherein the water content of the first solution is about 0 to about 10% of the total volume.

11. The method of any one of Claims 1 to 10, further comprising adding at least one pharmaceutical substance to either the first or the second solution, or to both.

12. The method of Claim 11, wherein the at least one pharmaceutical substance is selected from the group consisting of a protein, a peptide, an antibody, an antibody fragment, a biological, a tumor antigen, and a mixture thereof.

13. The method of Claim 12, wherein said peptide is a lipopeptide, a glycopeptide, or a glycolipopeptide.

14. The method of Claim 12, wherein said biological is selected from the group consisting of DNAs, RNAs, nucleosides, nucleotides, oligonucleotides, polynucleotides, synthetic organic molecules, natural organic molecules, vitamins, steroids, immunomodulators, cytokines, lymphokines, costimulatory molecules, antibiotics, antitumor agents, anti-inflammatory agents, anti-angiogenesis agents, chemokines, adjuvants, drugs acting on the central nervous system, an infectious disease agent antigen, and a mixture thereof.

15. The method of Claim 12, wherein the biological is interleukin-2.

16. The method of Claim 12, wherein the biological is an infectious disease agent antigen selected from the group consisting of a bacteria, a virus, a parasite, and a mixture thereof.

17. The method of Claim 12, wherein the tumor antigen is a peptide.

18. The method of Claim 17, wherein the peptide is a MUC-1 peptide or a derivative thereof.

19. The method of Claim 18, wherein the MUC-1 peptide is glycosylated.

20. The method of claim 18, wherein the MUC-1 peptide is BP1-148, having the following sequence:
NH₂-[STAPPAHGVTSAPDTRPAPGSTAPP(K-palmitoyl)G]-COOH.

21. The method according to any of claims 12, 17, 18, 19 or 20 wherein the liposome produced comprises a tumor antigen and said liposome is suitable for the manufacture of cancer vaccine.

22. The method of claim 21, wherein the lipsome further comprises at least one phospholipid, cholesterol, lipid A (natural or synthetic).

23. The method of claim 12 wherein the liposome produced comprises an infectious disease agent antigen and said liposome is suitable for the manufacture of an infectious disease agent vaccine.

24. The method of claims 23, wherein the infectious disease agent antigen is selected from the group consisting of a bacteria, a virus, a parasite, and a mixture thereof.

## Patentansprüche

1. Verfahren zum Herstellen eines Liposoms mit Mikrometergröße, wobei das Verfahren umfaßt:
(a) Bereitstellen einer ersten Lösung, welche eine Lipidkomponente in einem mit Wasser mischbaren Alkohol oder einem Alkohol/Wasser-Gemisch umfaßt, wobei die Lipidkonzentration der ersten Lösung wenigstens 10 mg/ml beträgt,
(b) Bereitstellen einer wäßrigen zweiten Lösung von wasserlöslichem Polymer, wobei das Polymer Polyethylenglycol (PEG) ist und die Polymerkonzentration wenigstens 25% (w/w) beträgt,
(c) Infundieren der ersten Lösung direkt in die zweite Lösung, wodurch eine Liposomsuspension gebildet wird, und
(d) Entfernen des Alkohols und des Polymers aus der Suspension, um das gewünschte Liposom mit Mikrometergröße zu erzeugen.

2. Verfahren nach Anspruch 1, wobei das erzeugte Liposom eine mittlere Größe von einem µm (Mikrometer) bis acht µm (Mikrometern) hat.

3. Verfahren nach Anspruch 1 oder 2, wobei der Alkohol und das Polymer durch Waschen des Gemischs mittels Zentrifugation oder Diafiltration entfernt werden.

4. Verfahren nach einem der Ansprüche 1 bis 3, wobei die Lipidkomponente aus der Gruppe ausgewählt ist, bestehend aus einem Phospholipid, einem Öl und einem Gemisch von gesättigten und ungesättigten Lipiden.

5. Verfahren nach Anspruch 4, wobei das Phospholipid aus der Gruppe ausgewählt ist, bestehend aus Phosphatidylcholin, Phosphatidylglycerol, Phosphatidsäure, Phosphatidylserin und Phosphatidylethanolamin, Dipalmitoylphosphatidylcholin, Distearoylphosphatidylcholin, Dimyristoylphosphatidylcholin, Dipalmitoylphosphatidylglycerol, Distearoylphosphatidylglycerol, Dimyristoylphosphatidylglycerol, Dipalmitoylphosphatidsäure, Dimyristoylphosphatidsäure, Distearoylphosphatidsäure, Dipalmitoylphosphatidylserin, Dimyristoylphosphatidylserin, Distearoylphosphatidylserin, Dipalmitoylphosphatidylethanolamin, Dimyristoylphosphatidylethanolamin und Distearoylphosphatidylethanolamin.

6. Verfahren nach einem der Ansprüche 1 bis 5, wobei die Lipidkomponente wenigstens ein Lipid und ein Sterol umfaßt.

7. Verfahren nach Anspruch 6, wobei das Sterol ein Cholesterol ist.

8. Verfahren nach einem der Ansprüche 1 bis 7, wobei das Lipid und der mit Wasser mischbare Alkohol oder das Lipid und das Alkohol/Wasser-Gemisch eine Emulsion darstellen.

9. Verfahren nach einem der Ansprüche 1 bis 8, wobei das Molekulargewicht des Polymers etwa 6.000 Dalton bis etwa 20.000 Dalton beträgt.

10. Verfahren nach einem der Ansprüche 1 bis 9, wobei der Wassergehalt der ersten Lösung etwa 0 bis etwa 10% des Gesamtvolumens ausmacht.

11. Verfahren nach einem der Ansprüche 1 bis 10, welches weiterhin die Zugabe wenigstens einer pharmazeutischen Substanz entweder zu der ersten oder zu der zweiten Lösung oder zu beiden umfaßt.

12. Verfahren nach Anspruch 11, wobei die wenigstens eine pharmazeutische Substanz aus der Gruppe ausgewählt ist, bestehend aus einem Protein, einem Peptid, einem Antikörper, einem Antikörperfragment, einem biologischen Präparat, einem Tumorantigen und einem Gemisch davon.

13. Verfahren nach Anspruch 12, wobei das Peptid ein Lipopeptid, ein Glycopeptid oder ein Glycolipopeptid ist.

14. Verfahren nach Anspruch 12, wobei das biologische Präparat aus der Gruppe ausgewählt ist, bestehend aus DNAs, RNAs, Nukleosiden, Nukleotiden, Oligonukleotiden, Polynukleotiden, synthetischen organischen Molekülen, natürlichen organischen Molekülen, Vitaminen, Steroiden, Immunmodulatoren, Zytokinen, Lymphokinen, costimulatorischen Molekülen, Antibiotika, Antitumormitteln, antiinflammatorischen Mitteln, gegen Angiogenese wirkenden Mitteln, Chemokinen, Adjuvanzien, Arzneimitteln, die auf das zentrale Nervensystem wirken, einem Antigen gegen einen Erreger von Infektionskrankheiten und einem Gemisch davon.

15. Verfahren nach Anspruch 12, wobei das biologische Präparat Interleukin-2 ist.

16. Verfahren nach Anspruch 12, wobei das biologische Präparat ein Antigen gegen einen Erreger von Infektionskrankheiten ist, ausgewählt aus der Gruppe, bestehend aus einem Bakterium, einem Virus, einem Parasiten und einem Gemisch davon.

17. Verfahren nach Anspruch 12, wobei das Tumorantigen ein Peptid ist.

18. Verfahren nach Anspruch 17, wobei das Peptid ein MUC-1-Peptid oder ein Derivat davon ist.

19. Verfahren nach Anspruch 18, wobei das MUC-1-Peptid glycosyliert ist.

20. Verfahren nach Anspruch 18, wobei das MUC-1-Peptid BP1-148 ist und die folgende Sequenz hat:
NH₂-[STAPPAHGVTSAPDTRPAPGSTAPP(K-Palmitoyl)G]-COOH.

21. Verfahren nach einem der Ansprüche 12, 17, 18, 19 oder 20, wobei das erzeugte Liposom ein Tumorantigen umfaßt und das Liposom für die Herstellung eines Krebsimpfstoffs geeignet ist.

22. Verfahren nach Anspruch 21, wobei das Liposom weiterhin wenigstens ein Phospholipid, Cholesterol oder Lipid A (natürlich oder synthetisch) umfaßt.

23. Verfahren nach Anspruch 12, wobei das erzeugte Liposom ein Antigen gegen einen Erreger von Infektionskrankheiten umfaßt und das Liposom für die Herstellung eines Impfstoffs gegen einen Erreger von Infektionskrankheiten geeignet ist.

24. Verfahren nach Anspruch 23, wobei das Antigen gegen einen Erreger von Infektionskrankheiten aus der Gruppe ausgewählt ist, bestehend aus einem Bakterium, einem Virus, einem Parasiten und einem Gemisch davon.

## Revendications

1. Procédé pour la préparation d'un liposome de dimensions égales aux micromètres, comprenant les étapes consistant à :
(a) fournir une première solution comprenant un constituant lipidique dans un alcool miscible à l'eau ou un mélange alcool/eau, la concentration en lipide de la première solution étant égale à au moins 10 mg/ml,
(b) fournir une seconde solution aqueuse d'un polymère hydrosoluble, dans laquelle le polymère est le polyéthylène-glycol (PEG) et la concentration en polymère est égale à au moins 25 % (en poids/poids),
(c) instiller la première solution directement dans la seconde solution, en formant ainsi une suspension de liposomes, et
(d) éliminer l'alcool et le polymère de la suspension pour produire le liposome désiré de dimensions égales aux micromètres.

2. Procédé suivant la revendication 1, dans lequel le liposome produit a une dimension moyenne d'un µm (micromètre) à huit µm (micromètres).

3. Procédé suivant la revendication 1 ou 2, dans lequel ledit alcool et ledit polymère sont éliminés par lavage du mélange par centrifugation ou diafiltration.

4. Procédé suivant l'une quelconque des revendications 1 à 3, dans lequel ledit constituant lipidique est choisi dans le groupe consistant en un phospholipide, une huile et un mélange de lipides saturés et insaturés.

5. Procédé suivant la revendication 4, dans lequel le phospholipide est choisi dans le groupe consistant en la phosphatidylcholine, le phosphatidylglycérol, l'acide phosphatidique, la phosphatidylsérine et la phosphatidyléthanolamine, la dipalmitoylphosphatidylcholine, la distéaroylphosphatidylcholine, la dimyristoylphosphatidylcholine, le dipalmitoylphosphatidylglycérol, le distéaroylphosphatidylglycérol, le dimyristoylphosphatidylglycérol, l'acide dipalmitoylphosphatidique, l'acide dimyristoylphosphatidique, l'acide distéaroylphosphatidique, la dipalmitoylphosphatidylsérine, la dimyristoylphosphatidylsérine, la distéaroylphosphatidylsérine, la dipalmitoylphosphatidyléthanolamine, la dimyristoylphosphatidyléthanolamine et la distéaroylphosphatidyléthanolamine.

6. Procédé suivant l'une quelconque des revendications 1 à 5, dans lequel ledit constituant lipidique comprend au moins un lipide et un stérol.

7. Procédé suivant la revendication 6, dans lequel le stérol est un cholestérol.

8. Procédé suivant l'une quelconque des revendications 1 à 7, dans lequel le lipide et l'alcool miscible à l'eau ou bien le lipide et le mélange alcool/eau sont présents sous forme d'une émulsion.

9. Procédé suivant l'une quelconque des revendications 1 à 8, dans lequel le poids moléculaire du polymère est d'environ 6000 daltons à environ 20 000 daltons.

10. Procédé suivant l'une quelconque des revendications 1 à 9, dans lequel le teneur en eau de la première solution est d'environ 0 à environ 10 % du volume total.

11. Procédé suivant l'une quelconque des revendications 1 à 10, comprenant en outre l'addition d'au moins une substance pharmaceutique à la première solution ou à la seconde solution, ou bien aux deux solutions.

12. Procédé suivant la revendication 11, dans lequel ladite au moins une substance pharmaceutique est choisie dans le groupe consistant en une protéine, un peptide, un anticorps, un fragment d'anticorps, un agent biologique, un antigène tumoral et un de leurs mélanges.

13. Procédé suivant la revendication 12, dans lequel ledit peptide est un lipopeptide, un glycopeptide ou un glycolipopeptide.

14. Procédé suivant la revendication 12, dans lequel ledit agent biologique est choisi dans le groupe consistant en des ADN, des ARN, des nucléosides, des nucléotides, des oligonucléotides, des polynucléotides, des molécules organiques synthétiques, des molécules organiques naturelles, des vitamines, des stéroïdes, des immunomodulateurs, des cytokines, des lymphokines, des molécules costimulatrices, des antibiotiques, des agents antitumoraux, des agents anti-inflammatoires, des agents antiangiogenèse, des chimiokines, des adjuvants, des médicaments agissant sur le système nerveux central, un antigène d'un agent d'une maladie infectieuse et un de leur mélanges.

15. Procédé suivant la revendication 12, dans lequel l'agent biologique est l'interleukine-2.

16. Procédé suivant la revendication 12, dans lequel l'agent biologique est un antigène d'agent de maladies infectieuses choisi dans le groupe consistant en une bactérie, un virus, un parasite et un de leurs mélanges.

17. Procédé suivant la revendication 12, dans lequel l'antigène tumoral est un peptide.

18. Procédé suivant la revendication 17, dans lequel le peptide est un peptide MUC-1 ou un de ses dérivés.

19. Procédé suivant la revendication 18, dans lequel le peptide MUC-1 est glycosylé.

20. Procédé suivant la revendication 18, dans lequel le peptide MUC-1 est le BP1-148, ayant la séquence suivante :
NH₂-[STAPPAHGVTSAPDTRPAPGSTAPP(K-palmitoyl)G]-COOH.

21. Procédé suivant l'une quelconque des revendications 12, 17, 18, 19 ou 20, dans lequel le liposome produit comprend un antigène tumoral et ledit liposome est apte à la production d'un vaccin contre le cancer.

22. Procédé suivant la revendication 21, dans lequel le liposome comprend en outre au moins un phospholipide, du cholestérol, le lipide A (naturel ou synthétique).

23. Procédé suivant la revendication 12, dans lequel le liposome produit comprend un antigène d'agent de maladie infectieuse et ledit liposome est apte à la production d'un vaccin contre l'agent de maladie infectieuse.

24. Procédé suivant la revendication 23, dans lequel l'antigène d'agent de maladie infectieuse est choisi dans le groupe consistant en une bactérie, un virus, un parasite et un de leurs mélanges.
